# EUROPÄISCHE PATENTSCHRIFT

(11) **EP 0 746 276 B1**
(45) Veröffentlichungstag und Bekanntmachung des Hinweises auf die Patenterteilung: **05.08.1998**
(21) Anmeldenummer: 95911258.2
(22) Anmeldetag: 23.02.1995
(51) Int. Cl.: A61F 2/42, A61F 2/30

(54) **HANDGELENKPROTHESE**
HAND JOINT PROSTHESIS
PROTHESE DU POIGNET

(30) Priorität: 24.02.1994 DE 4406090
(43) Veröffentlichungstag der Anmeldung: 11.12.1996
(73) Patentinhaber: PLUS ENDOPROTHETIK AG, 6343 Rotkreuz (CH)
(72) Erfinder: SIMMEN, Beat, R., Dr. med., CH-8307 Tagelswangen (CH); Bähler, André, CH-8032 Zürich (CH)
(74) Vertreter: Popp, Eugen, Dr.
(86) Internationale Anmeldenummer: EP9500664
(87) Internationale Veröffentlichungsnummer: WO9522945

(56) Entgegenhaltungen:
- EP-A- 0 342 014
- EP-A- 0 532 440
- WO-A-83/02555
- DE-U- 9 316 763
- US-A- 4 229 841
- US-A- 4 307 473
- US-A- 4 714 476

## Beschreibung

Die Erfindung betrifft eine Handgelenkprothese mit
- einem radialen Teil, das einen im distalen Bereich des radialen Markkanals aufnehmbaren und darin befestigbaren Schaft aufweist,
- einem metakarpalen Teil, das zwei Schäfte aufweist, die sich distal erstrecken, und von denen der eine in den proximalen Bereich des dritten Mittelhandknochens aufnehmbar und darin befestigbar ist, während der andere im Bereich der Handwurzel verankerbar ist, und mit
- einer Gelenkverbindung zwischen dem radialen und dem metakarpalen Teil, wobei
- die Gelenkverbindung (15) derart ist, daß es eine dorsal/palmare Flexion einerseits und eine radio-ulnare Duktion andererseits gestattet, wobei der radio-ulnare Schwenkradius (R₁) größer ist als der dorsal/ palmare Schwenkradius (R₂).

Eine derartige Handgelenkprothese ist aus der US-A-4 714 476 bekannt. Die in der Praxis gängigsten Konstruktionen sind in der US-A-4 784 661 einerseits und in der US-A-4 106 128 bzw. US-A-4 063 314 bzw. US-A-4 180 871 andererseits beschrieben. Die letztgenannten Druckschriften betreffen jeweils Handgelenkprothesen mit einer Kugelgelenkverbindung Zwischen dem radialen und dem metakarpalen Teil. Dementsprechend erlauben diese Handprothesen sowohl eine dorsal-palmare Flexion als auch radio-ulnare Duktion sowie axiale Rotation. Der Nachteil dieser Gelenkverbindung ist die mangelnde Stabilität. Man spricht von einem sogenannten "unconstrained" Gelenk. Die Kugelgelenkverbindung wäre an sich ideal; denn sie kommt dem natürlichen Handgelenk am nächsten. Wird jedoch bei der Implantation der mechanische Mittelpunkt der Kugelgelenkverbindung in Verbindung mit den im Handgelenk vorhandenen Sehnen nicht gefunden, erfolgt eine einseitige Belastung der Gelenkverbindung mit der Folge von Fehlstellungen und eines entsprechenden Abriebs. Dieser wird vor allem dann deutlich, wenn als Lagermaterial Polyethylen verwendet wird. Aus diesem Grunde ist die Lebensdauer einer derartigen Handgelenkprothese sehr begrenzt.

Die Handgelenkprothese gemäß der US-A-4 784 661 weist eine Gelenkverbindung zwischen dem radialen und metakarpalen Teil mit einem ellipsoiden Gelenkelement am metakarpalen Teil und einer komplementären Lagerfläche am radialen Teil auf. Dieser Handgelenkprothese wird mittlerweile gegenüber der Handprothese mit Kugelgelenkverbindung der Vorzug gegeben. Problematisch ist jedoch bei der Konstruktion nach der US-A-4 784 661, daß eine axiale Rotation nicht ausgeschlossen ist mit der Folge, daß dann das ellipsoide Lagerelement von der komplementären Lagerfläche abhebt. Das gleiche gilt für Winkelbewegungen außerhalb der Achsen für Flexion-Extension und Radial-Ulnarduktion. Dies führt zu frühzeitigem Verschleiß der Lagerflächen. Auch eine Luxation ist nicht ausgeschlossen.

Der vorliegenden Erfindung liegt die Aufgabe zugrunde, eine Handgelenkprothese der eingangs genannten Art zu schaffen, die durch eine einfache und verschleißarme Gelenkverbindung zwischen dem radialen und metakarpalen Teil gekennzeichnet ist, wobei diese Gelenkverbindung hinsichtlich der Beweglichkeit dem anatomischen Handgelenk weitgehend entspricht.

Die vorgenannte Erfindung wird durch die kennzeichnenden Merkmale des Anspruches 1 gelöst. Die erfindungsgemäße Handgelenkprothese erlaubt also keine axiale Rotation zwischen radialem und metakarpalem Teil. Diese Rotation ist auf den Unterarm verlagert. Es hat sich gezeigt, daß für das Handgelenk selbst keine axiale Rotation erforderlich ist. Dieser Erkenntnis entsprechend kann die Gelenkverbindung zwischen radialem und metakarpalem Teil sehr einfach gebaut werden, insbesondere auch wesentlich einfacher als bei dem Handgelenk gemäß der US-A-4 307 473, welches ebenfalls eine Kugelgelenkverbindung beinhaltet. Darüber hinaus wird durch die erfindungsgemäße Wahl der Schwenkradien eine starke Annäherung an das anatomische Handgelenk erreicht. In diesem Zusammenhang ist zu bedenken, daß die in den bindegewebigen Halteapparat eingeleiteten Kräfte pro Winkelgrad für "dorsal/palmar" wesentlich geringer sind als für "radial/ulnar". Dieser Erkenntnis entsprechend ist bei der erfindungsgemäßen Handgelenkprothese der radio-ulnare Schwenkradius deutlich größer als der dorsal/palmare Schwenkradius. Es wird dazu auch auf Anspruch 2 verwiesen. Vorzugsweise beträgt der radio-ulnare Schwenkradius etwa 15 bis 30 mm, insbesondere 15 bis 20 mm, während der dorsal/palmare Schwenkradius nur etwa 2 bis 5 mm, insbesondere 2 bis 3 mm beträgt. Durch die erfindungsgemäß gewählten Schwenkradien hat die Gelenkverbindung eine geringere Tendenz zur Destabilisierung.

Eine selbstzentrierende Funktion wird durch die Maßnahmen gemäß Anspruch 5 erreicht. Bei zunehmender radio-ulnarer Winkelbildung wird eine entsprechend zunehmende Rückstellkraft auf die Gelenkverbindung ausgeübt. Dadurch erhält man die erwähnte Selbstzentrierung der Gelenkverbindung in radioulnarer Ebene.

Bevorzugte konstruktive Details sind in den Unteransprüchen beschrieben. Die erfindungsgemäße Gelenkverbindung kommt sowohl bei radio-ulnarer als auch dorso-palmarer Winkelbildung ohne Anschlag aus. Dementsprechend groß sind die beiden Winkelbereiche.

Des weiteren zeichnet sich die konkrete Konstruktion gemäß Anspruch 3 dadurch aus, daß die sich berührenden Gelenkflächen in allen Relativstellungen zueinander stets kongruent sind. Dies ist nicht der Fall bei der derzeit bevorzugt implantierten Handgelenkprothese gemäß der US-A-4 784 661, wie die eingangs dazu gemachten Erläuterungen erkennen lassen. Bei axialer Rotation und bei Winkelbewegungen außerhalb der Ebenen für Flexion-Extension und Radial-Ulnarduktion heben nämlich die ellipsoiden Lagerflächen voneinander ab. Die Kongruenz der Lager- bzw. Gelenkflächen geht dabei verloren mit der Folge eines erhöhten Verschleißes bis hin zur Lockerung der Prothese.

Vorteilhaft ist bei der erfindungsgemäßen Konstruktion auch der gesonderte Kragen gemäß den Ansprüchen 7 bis 9. Vorzugsweise wird ein Satz von Kragen unterschiedlicher Größe zur Verfügung gestellt entsprechend den verschiedenen Gelenkgrößen bzw. Knochendimensionen.

Die Handgelenkprothese gemäß Erfindung besteht für die zementfreie Implantation vorzugsweise aus Titan, wobei entsprechend Anspruch 4 die Lageröffnung für die kreissegmentartige Gelenkscheibe dann vorzugsweise Teil eine Polyethylen- oder Keramikeinsatzes ist. Auch ist ein Lagermetalleinsatz denkbar.

Die kreissegmentartige Gelenkscheibe kann statt aus Titan auch aus Keramik oder Stahl hergestellt sein. Grundsätzlich ist es auch denkbar, die gesamte Handgelenkprothese aus Keramik oder Stahl herzustellen. Dies hätte den Vorteil, daß die Handgelenkprothese sowohl für eine zementfreie Implantation als auch Implantation mit Zement geeignet ist.

Die Oberfläche des Kragens ist vorzugsweise aufgerauht. Über den Umfang kann der Kragen zusätzlich längsgerippt ausgebildet sein, um zwischen der Corticalis und dem Kragen Spongiosa-Freiräume zu erhalten und ein Heranwachsen des Knochengewebes an die Implantatoberfläche zu ermöglichen (Bony Ingrowth).

Nachstehend wird eine Ausführungsform einer erfindungsgemäß ausgebildeten Handgelenkprothese anhand der beigefügten Zeichnung näher erläutert. Es zeigen:
- Fig. 1: einen schematischen Längsschnitt durch eine Handgelenkprothese gemäß Erfindung;
- Fig. 2: das metakarpale Teil der erfindungsgemäßen Handgelenkprothese in Draufsicht;
- Fig. 3: das radiale Teil der erfindungsgemäßen Handgelenkprothese in Draufsicht;
- Fig. 4: einen in den distalen Bereich des radialen Markkanals einsetzbaren Kragen in Draufsicht;
- Fig. 5: den Kragen gemäß Fig. 4 in Unteransicht;
- Fig. 6: das metakarpale Gelenkteil in schematischem Längs-schnitt mit einer etwa U-förmigen Gleitlagerfläche; und
- Fig. 7: eine abgewandelte Ausführungsform eines Kragens entsprechend den Fig. 4 und 5 in perspektivischer Draufsicht.

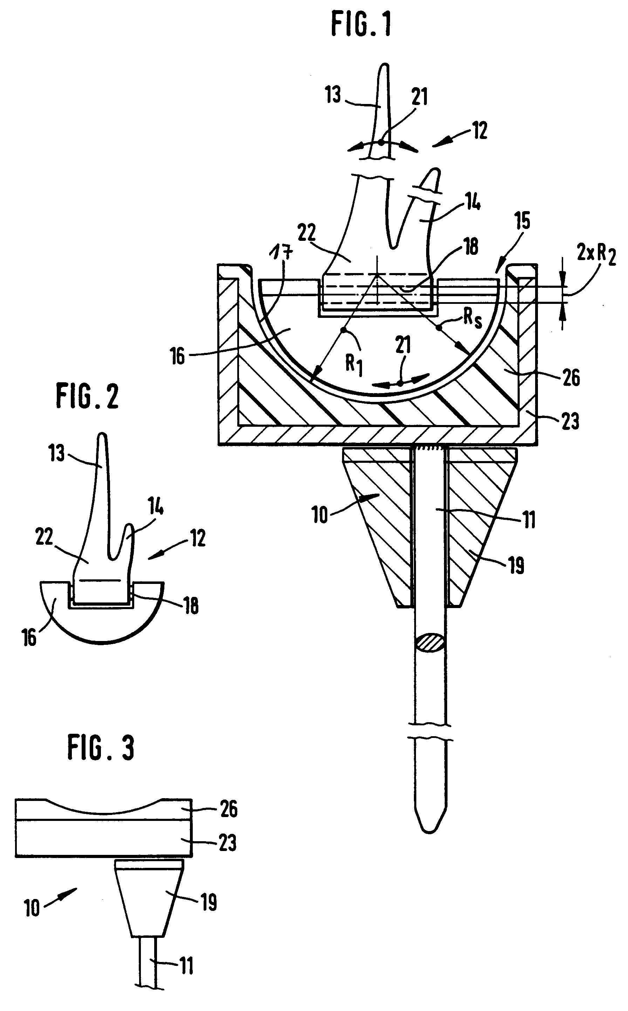

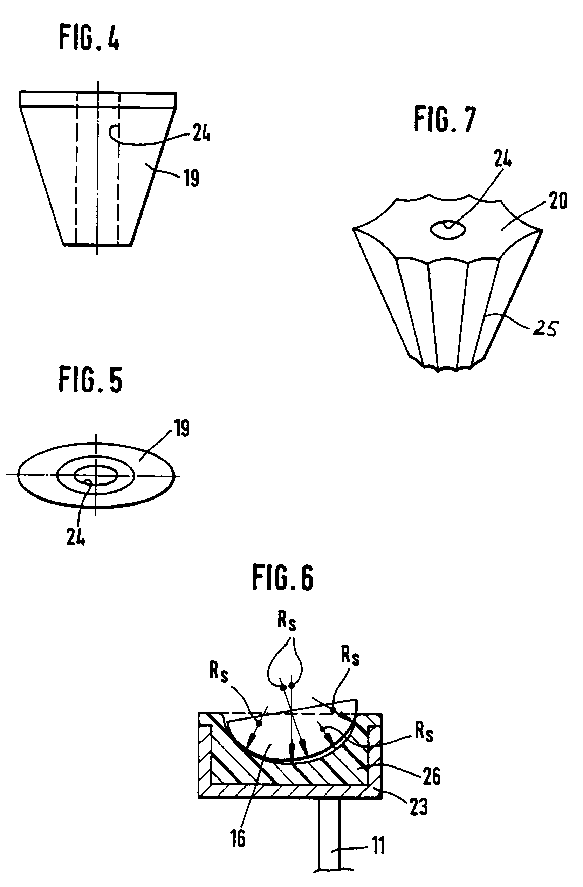

In Fig. 1 ist in schematischem Längsschnitt eine bevorzugte Ausführungsform für eine Handgelenkprothese dargestellt mit einem radialen Teil 10, das einen im distalen Bereich des radialen Markkanals (hier nicht dargestellt) aufnehmbaren und darin befestigbaren Schaft 11 aufweist. Dieser Schaft 11 ist stiftartig ausgebildet und weist einen flachovalen Querschnitt auf. Dieser Querschnitt korrespondiert mit einer entsprechenden Axialbohrung durch einen über den Schaft 11 schiebbaren Kragen entsprechend den Fig. 4, 5 bzw. 7, welcher im distalen Bereich des radialen Markkanals plazierbar ist. Der vorgenannte Kragen hat die Bezugsziffer 19 bzw. 20.

Des weiteren umfaßt die Handgelenkprothese gemäß Fig. 1 ein metakarpales Teil 12, das zwei unterschiedlich lange Schäfte 13 und 14 aufweist, die sich distal erstrecken, wobei der längere Schaft in den proximalen Bereich des dritten Mittelhandknochens aufnehmbar und darin befestigbar ist. Der kürzere Schaft wird in der Handwurzel (vorzugsweise im Kahnbein) verankert. Diesbezüglich handelt es sich um ein an sich bekanntes Design. Zwischen dem metakarpalen Teil 12 und dem radialen Teil 10 ist eine Gelenkverbindung 15 angeordnet. Diese ist biaxial derart, daß es eine dorsal/palmare Flexion einerseits und eine radio-ulnare Duktion entsprechend Doppelpfeil 21 andererseits gestattet. Die Konstruktion ist so ausgebildet, daß der radio-ulnare Schwenkradius R₁ größer ist. als der dorsal/palmare Schwenkradius R₂. Die Größenverhältnisse von R₁ und R₂ sind eingangs erwähnt. Vorzugsweise ist der radio-ulnare Schwenkradius R₁ etwa 5 bis 15mal, insbesondere etwa 10mal größer als der dorsal/palmare Schwenkradius R₂. Der radio-ulnare Schwenkradius R₁ ist durch das Zusammenspiel einer kreissegmentartigen Gelenkscheibe 16 am proximalen Ende des metakarpalen Teils 12 mit einer komplementären Lageröffnung 17 am distalen Ende des radialen Teils 10 definiert. Entsprechend der kreissegmentartigen Gelenkscheibe 16 ist die komplementäre Lageröffnung 17 eine Nut mit etwa kreisförmigem Boden.

Die beiden metakarpalen Schäfte 13 und 14 sind über ein flaches Verbindungselement 22 an der kreissegmentartigen Gelenkscheibe 16 angelenkt, wobei die sich von "radial" nach "ulnar" erstreckende Gelenkachse 18 den dorsal/palmaren Schwenkradius R₂ definiert. Der halbe Durchmesser der Gelenkachse 18 stellt also den dorsal/palmaren Schwenkradius R₂ dar.

Die erwähnte Lageröffnung 17 zur Aufnahme der kreissegmentartigen Gelenkscheibe 16 ist bei der dargestellten Ausführungsform Teil eines Polyethyleneinsatzes, der in eine topfartige Aufnahme aus Metall, insbesondere Titan bzw. Titanlegierung, einpaßbar ist. Am Boden des Aufnahmetopfes 23 ist der bereits erwähnte Schaft 11 befestigt.

Entsprechend Fig. 6 kann der Radius R_{S} der kreissegmentartigen Gelenkscheibe 16 und korrespondierende Radius der komplementären Lageröffnung 17 randseitig kleiner sein als im Zentralbereich, insbesondere zu den beiden Rändern hin kontinuierlich kleiner werden, so daß eine etwa U-förmige Gleitflächenkontur von Gelenkscheibe 16 und Lageröffnung 17 entsteht. Dadurch wird die eingangs erwähnte Selbstzentrierung der Gelenkverbindung in radio-ulnarer Ebene erhalten.

Des weiteren lassen sowohl Fig. 1 als auch Fig. 6 erkennen, daß die Gelenkverbindung 15 gegenüber der radialen Schaftachse 11 in ulnarer Richtung versetzt angeordnet ist.

Auf den am radialen Teil 10 angeordneten Schaft 11 ist entsprechend Fig. 3 ein Kragen 19 aufschiebbar, der im implantierten Zustand des radialen Teils innerhalb des distalen Bereiches des radialen Markkanals plazierbar ist. Der Kragen 19 verjüngt sich von distal nach proximal konisch. Entsprechend Fig. 5 ist er im Querschnitt flach oval ausgebildet. Das gleiche gilt für die axiale Durchgangsbohrung 24 entsprechend dem flach ovalen Querschnitt des Schaftes 11. Damit kann der Kragen drehsicher am Schaft 11 plaziert werden, und zwar in Anlage am Boden des Aufnahmetopfes 23.

Entsprechend Fig. 7 kann der Kragen 20 über seinen Umfang längsgerippt ausgebildet sein. Die Längsrippen sind in Fig. 7 mit der Bezugsziffer 25 gekennzeichnet. Damit werden zwischen Corticalis und Kragen 20 Spongiosa-Freiräume geschaffen, die die Versorgung des Knochens bis zum distalen Bereich hin sicherstellen und ein Heran- und Einwachsen des Knochengewebes bis an die Implantatoberfläche gestatten.

Die Länge des längeren Schaftes 13 am metakarpalen Teil 12 beträgt etwa 35 bis 45, insbesondere etwa 40 mm. Die Anlenkung der Schäfte 13 und 14 an der Gelenkscheibe 16 erlaubt eine Dorsalneigung von etwa 7 bis 10, insbesondere etwa 8°.

Die beiden Schäfte 13 und 14 erstrecken sich etwa parallel zueinander. Der kleinere Schaft 14 hat eine Länge von etwa 12 bis 20, insbesondere etwa 15 mm.

Die Länge des Aufnahmetopfes 23 beträgt etwa 30 bis 35 mm. Er weist eine Breite von etwa 12 bis 20 mm, insbesondere etwa 15 bis 16 mm auf. Die Querschnittsabmessung des Schaftes 11 beträgt etwa 3 x 4 mm. Der Schaft 11 ist vorzugsweise gegenüber der geometrischen Mittenachse nicht nur nach radial, sondern auch noch nach dorsal versetzt.

Der Querschnitt der Gelenkscheibe 16 und dementsprechend auch der Lageröffnung 17 ist vorzugsweise rechteckförmig, wobei die innenliegenden Kanten mehr oder weniger stark abgerundet sein können.

Der Kragen 19 bzw. 20 weist eine Axialerstreckung von etwa 17 bis 25 mm, insbesondere etwa 20 mm auf. Die Länge des Schaftes 11 beträgt etwa 40 bis 50, insbesondere etwa 45 mm. Die Höhe des Aufnahmetopfes 23 beträgt etwa 10 bis 15, insbesondere etwa 12 mm. Die minimale Wandstärke des PolyethylenEinsatzes bzw. Inlays innerhalb des Aufnahmetopfes 23 beträgt etwa 2 bis 5 mm.

Die vorgenannten Abmessungen sind Durchschnittswerte, von denen in Einzelfällen abgewichen werden kann. Letztlich hängen die Abmessungen von der Größe des Handgelenks ab, in welches die beschrieben Prothese implantiert werden soll.

### Bezugszeichenliste

- 10: Radialer Teil
- 11: Schaft
- 12: metakarpaler Teil
- 13: Schaft
- 14: Schaft
- 15: Gelenkverbindung
- 16: kreissegmentartige Gelenkscheibe
- 17: Lageröffnung
- 18: Gelenkachse
- 19: Kragen
- 20: Kragen
- 21: Doppelpfeil
- 22: Verbindungselement
- 23: Aufnahmetopf
- 24: Bohrung
- 25: Längsrippe
- 26: Polyethylen-Einsatz

## Patentansprüche

1. Handgelenkprothese mit
- einem radialen Teil (10), das einen im distalen Bereich des radialen Markkanals aufnehmbaren und darin befestigbaren Schaft (11) aufweist,
- einem metakarpalen Teil (12), das zwei Schäfte (13, 14) aufweist, die sich distal erstrecken, und von denen der eine in den proximalen Bereich des dritten Mittelhandknochens aufnehmbar und darin befestigbar ist, wahrend der andere im Bereich der Handwurzel verankerbar ist, und mit
- einer Gelenkverbindung (15) zwischen dem radialen und dem metakarpalem Teil, wobei
- die Gelenkverbindung (15) derart ist, daß es eine dorsal/palmare Flexion einerseits und eine radio-ulnare Duktion andererseits gestattet, wobei der radio-ulnare Schwenkradius (R₁) größer ist als der dorsal/ palmare Schwenkradius (R₂).
**dadurch gekennzeichnet,** daß
die Gelenkverbindung (15) biaxial ist, wobei der radio-ulnare Schwenkradius (R₁) durch das Zusammenspiel einer kreissegmentartigen Gelenkscheibe (16) am proximalen Ende des metakarpalen Teils (12) mit einer komplementären Lageröffnung (17) am distalen Ende des radialen Teils (10) definiert ist.

2. Handgelenkprothes nach Anspruch 1,
dadurch gekennzeichnet, daß der radio-ulnare Schwenkradius (R₁) etwa 5 bis 15mal, insbesondere etwa 10mal größer ist als der dorsal/palmare Schwenkradius (R₂)

3. Handgelenkprothese nach Anspruch 1 oder 2,
dadurch gekennzeichnet, daß die beiden metakarpalen Schäfte (13, 14) an der kreissegmentartigen Gelenkscheibe (16) angelenkt sind, wobei die sich von radial nach ulnar erstreckende Gelenkachse (18) den dorsal/ palmaren Schwenkradius (R₂) definiert.

4. Handgelenkprothese nach einem der Anspüche 1 bis 3,
dadurch gekennzeichnet, daß die Lageröffnung (17) für die kreissegmentartige Gelenkscheibe (16) Teil eines Polyethylen-, Keramik- oder Lagermetall-Einsatzes ist.

5. Handgelenkprothese nach einem der Ansprüche 1 bis 4,
dadurch gekennzeichnet, daß der Radius (R_{S}) der kreissegmentartigen Gelenkscheibe (16) und korrespondierende Radius der komplementären Lageröffnung (17) randseitig kleiner ist als im Zentralbereich, insbesondere zum radialen bzw. ulnaren Rand hin kontinuierlich kleiner wird.

6. Handgelenkprothese nach einem der Ansprüche 1 bis 5,
dadurch gekennzeichnet, daß die Gelenkverbindung (15) gegenüber der radialen Schaftachse (11) in ulnarer Richtung versetzt angeordnet ist.

7. Handgelenkprothese nach einem der Ansprüche 1 bis 6,
dadurch gekennzeichnet, daß auf den am radialen Teil (10) angeordneten Schaft (11) ein Kragen (19; 20) aufschiebbar ist.

8. Handgelenkprothese nach Anspruch 7,
dadurch gekennzeichnet, daß der Kragen (19; 20) sich von distal nach proximal konisch verjüngt, und im Querschnitt kreisrund, insbesondere aber flach oval ausgebildet ist.

9. Handgelenkprothese nach Anspruch 7 oder 8,
dadurch gekennzeichnet, daß der Kragen (20) über seinen Umfang längsgerippt (Längsrippen 25) ausgebildet ist.

## Claims

1. Hand joint prosthesis comprising
- a radial part (10) having a shaft (11) which is accommodated in the distal area of the radial marrow channel and fixed therein;
- a metacarpal part (12) having two shafts (13, 14), which extend distally and of which the one is accommodated and fixed in the proximal area of the third middle hand bone whereas the other is anchored in the area of the hand root; and
- with a joint connection (15) between the radial and the metacarpal part; and
- the joint connection (15) is such that it permits both dorsal/palmar flexion and radio-ulnar duction, and the radioulnar pivotal radius (R₁) is larger than the dorsal/palmar pivotal radius (R₂),
**characterised in that** the joint connection (15) is biaxial, and the radio-ulnar pivotal radius (R₁) is defined by co-action of a circular segmentlike joint disc (16) at the proximal end of the metacarpal part (12) with a complementary mounting aperture (17) at the distal end of the radial part (10).

2. Hand joint prosthesis according to Claim 1, **characterised in that** the radio-ulnar pivotal radius (R₁) is between 5 and 15 times, in particular approximately 10 times, larger than the dorsal/palmar pivotal radius (R₂).

3. Hand joint prosthesis according to Claim 1 or 2, **characterised in that** both metacarpal shafts (13, 14) are hinged onto the circular segmentlike joint disc (16), and the joint axis (18), which extends radially towards ulnar, defines the dorsal/palmar pivotal radius (R₂).

4. Hand joint prosthesis according to one of Claims 1 to 3, **characterised in that** the mounting aperture (17) for the circular segmentlike joint disc (16) is part of a polyethylene, ceramic or bearing metal insert.

5. Hand joint prosthesis according to one of Claims 1 to 4, **characterised in that** the radius (R_{S}) of the circular segmentlike joint disc (16) and the corresponding radius of the complementary mounting aperture (17) is at the edge smaller than in the central area, in particular continuously reducing towards the radial or ulnar edge.

6. Hand joint prosthesis according to one of Claims 1 to 5, **characterised in that** the joint connection (15) is arranged to be offset in the ulnar direction relative to the radial shaft axis (11).

7. Hand joint prosthesis according to one of Claims 1 to 6, **characterised in that** a collar (19; 20) can be pushed onto the shaft (11) which is arranged on the radial part (10).

8. Hand joint prosthesis according to Claim 7, **characterised in that** the collar (19; 20) narrows from distal to proximal and is cross-sectionally circular, in particular of flat oval shape.

9. Hand joint prosthesis according to Claim 7 or 8, **characterised in that** the collar (20) is longitudinally webbed (longitudinal webs 25) over its periphery.

## Revendications

1. Prothèse du poignet comportant
- une partie radiale (10) qui présente une queue (11) pouvant être logée et fixée dans la partie distale du canal médullaire radial,
- une partie métacarpienne (12) qui présente deux queues (13, 14) qui s'étendent distalement et dont l'une peut être logée et fixée dans la partie proximale du troisième métacarpien, tandis que l'autre peut être ancrée dans la région du carpe, et
- une articulation (15) entre la partie radiale et la partie métacarpienne,
- cette articulation (15) étant telle qu'elle permette d'une part une flexion dorsale/palmaire et d'autre part une duction radio-ulnaire, le rayon de rotation radio-ulnaire (R1) étant supérieur au rayon de rotation dorsal/palmaire (R2),
caractérisée par le fait que l'articulation (15) est biaxe, le rayon de rotation radio-ulnaire (R1) étant défini par la coopération d'un disque d'articulation du genre segment de cercle (16) situé à l'extrémité proximale de la partie métacarpienne (12) avec une ouverture complémentaire d'appui (17) située à l'extrémité distale de la partie radiale (10).

2. Prothèse du poignet selon la revendication 1, caractérisée par le fait que le rayon de rotation radio-ulnaire (R1) est environ 5 à 15 fois, en particulier environ 10 fois, plus grand que le rayon de rotation dorsal/palmaire (R2).

3. Prothèse du poignet selon l'une des revendications 1 et 2, caractérisée par le fait que les deux queues métacarpiennez (13, 14) sont articulées sur le disque d'articulation du genre segment de cercle (16), l'axe matériel d'articulation (18), s'étendant du côté radial vers le côté ulnaire, définissant le rayon de rotation dorsal/palmaire (R2).

4. Prothèse du poignet selon l'une des revendications 1 à 3, caractérisée par le fait que l'ouverture d'appui (17) pour le disque d'articulation du genre segment de cercle (16) fait partie d'un élément rapporté en polyéthylène, céramique ou métal antifriction.

5. Prothèse du poignet selon l'une des revendications 1 à 4, caractérisée par le fait que le rayon (RS) du disque d'articulation du genre segment de cercle (16) et le rayon correspondant de l'ouverture complémentaire d'appui (17) sont plus petits du côté du bord que dans la partie centrale, en particulier diminuent de manière continue vers le bord radial et le bord ulnaire.

6. Prothèse du poignet selon l'une des revendications 1 à 5, caractérisée par le fait que l'articulation (15) est décalée dans la direction ulnaire par rapport à la queue radiale (11).

7. Prothèse du poignet selon l'une des revendications 1 à 6, caractérisée par le fait qu'un col (19 ; 20) peut être glissé sur la queue (11) placée sur la partie radiale (10).

8. Prothèse du poignet selon la revendication 7, caractérisée par le fait que le col (19 ; 20) se rétrécit de manière conique du côté distal vers le côté proximal et est de section ronde, en particulier ovale plate.

9. Prothèse du poignet selon l'une des revendications 7 et 8, caractérisée par le fait que le col (20) est nervuré longitudinalement (nervures longitudinales 25) sur son pourtour.
